# EUROPÄISCHE PATENTANMELDUNG

(11) **EP 1 921 069 A1**
(43) Veröffentlichungstag der Anmeldung: **14.05.2008**
(21) Anmeldenummer: 06123705.3
(22) Anmeldetag: 08.11.2006
(51) Int. Cl.: C07D 231/56

(54) **Verfahren zur Herstellung von 2-Alkyl-3-heterocyclyl-prop-2-en-1-olen**

(71) Anmelder: Speedel Experimenta AG, 4123 Allschwil (CH)
(72) Erfinder: Die Erfindernennung liegt noch nicht vor
(74) Vertreter: Maué, Paul Georg

(57) **Zusammenfassung**

Verfahren zur Herstellung einer Verbindung der Formel I, 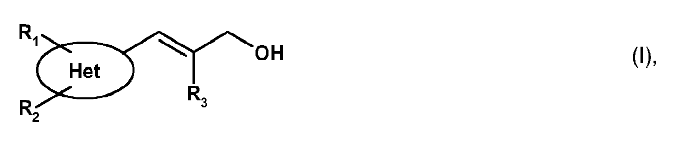 worin R₁ und R₂ unabhängig voneinander H, C₁-C₈-Alkyl, Halogen, Polyhalogen-C₁-C₈-alkoxy, Polyhalogen-C₁-C₈-alkyl, C₁-C₈-Alkoxy, C₁-C₈-Alkoxy-C₁-C₈-alkyl, oder C₁-C₈-Alkoxy-C₁-C₈-alkoxy darstellen, wobei R₁ und R₂ nicht gleichzeitig H bedeuten und R₃ C₁-C₈-Alkyl bedeutet, durch Reaktion von Prop-2-in-1-ol mit R₁- und R₂-substituierten ungesättigten, bicyclischen Heterocyclyl-Bromiden, -Iodiden, -Triflaten, -Tosylaten oder-Mesylaten der Formel R-X zu den entsprechenden 3-R-prop-2-in-1-olen gefolgt von der Addition einer Alkylmetallverbindung, worin "Alkyl" C₁-C₈-Alkyl darstellt und R für 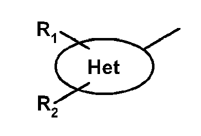 steht.

## Beschreibung

Die Erfindung betrifft ein stereoselektives Verfahren zur Herstellung von (E)-2-Alkyl-3-heterocyclyl-prop-2-en-1-ol und neuer Zwischenprodukte, die bei den Verfahrensstufen erhalten werden.

In der WO 2005/090305 A1 werden δ-Amino-γ-hydroxy-ω-heterocyclyl-alkancarbonsäureamide beschrieben, die Renin-hemmende Eigenschaften aufweisen und als antihypertensive Mittel in pharmazeutischen Zubereitungen verwendet werden können. Die dort beschriebenen Herstellungsverfahren, die über eine Kupplung einer Heterocyclyl-Metall-Spezies an einen Aldehyd als Schlüsselschritt verlaufen, sind insbesondere hinsichtlich der zum Teil unbefriedigenden Ausbeuten nicht für ein industrielles Verfahren geeignet.

In einem neuen Verfahren geht man von 2,7-Dialkyl-8-heterocyclyl-4-octenoylamiden aus, deren Doppelbindung gleichzeitig in 5-Stellung halogeniert und in 4-Stellung unter Lactonisierung hydroxyliert wird, dann das Halogen mit Azid ersetzt, das Lacton amidiert und darauf das Azid in die Amingruppe überführt wird. Die gewünschten Alkancarbonsäureamide werden bei diesem neuen Verfahren in wesentlich höheren Gesamtausbeuten erhalten. Die Halolaktonisierung, die Azidierung und die Azidreduktion werden nach dem von P. Herold im Journal of Organic Chemistry, Vol. 54 (1989), Seiten 1178-1185 beschriebenen Verfahren durchgeführt.

Die 2,7-Dialkyl-8-heterocyclyl-4-octenoylamide können zum Beispiel der Formel A entsprechen, 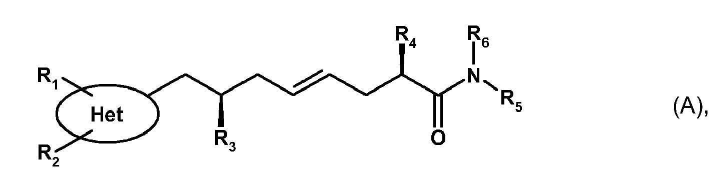 worin Het ein bicyclisches, über ein C-Atom an das Restmolekül geknüpftes, ungesättigtes Heterocyclyl bedeutet, wobei der nicht direkt an das Restmolekül gebundene Ring durch R₁ und R₂ substituiert ist, R₁ und R₂ unabhängig voneinander H, C₁-C₈-Alkyl, Halogen, Polyhalogen-C₁-C₈-alkoxy, Polyhalogen-C₁-C₈-alkyl, C₁-C₈-Alkoxy, C₁-C₈-Alkoxy-C₁-C₈-alkyl, oder C₁-C₈-Alkoxy-C₁-C₈-alkoxy darstellen, wobei R₁ und R₂ nicht gleichzeitig H bedeuten, R₃ C₁-C₈-Alkyl bedeutet, R₄ für C₁-C₈-Alkyl steht, R₅ C₁-C₈-Alkyl oder C₁-C₈-Alkoxy bedeutet, R₆ C₁-C₈-Alkyl darstellt, oder R₅ und R₆ zusammen gegebenenfalls mit C₁-C₄-Alkyl, Phenyl oder Benzyl substituiertes Tetramethylen, Pentamethylen, 3-Oxa-1,5-pentylen oder -CH₂CH₂O-C(O)- sind.

Die Verbindungen der Formel A sind erhältlich, indem man eine Verbindung der Formel B 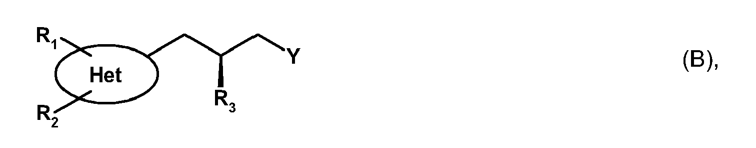 mit einer Verbindung der Formel C, 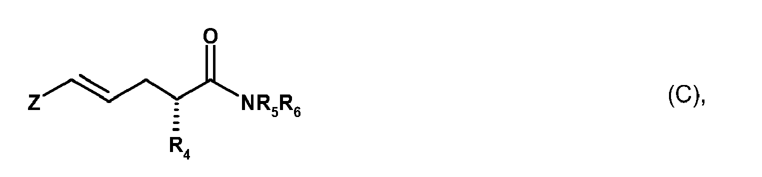 worin Het und R₁ bis R₆ die zuvor angegebenen Bedeutungen haben, Y Cl, Br oder I und Z Cl, Br oder I bedeuten, in Gegenwart eines Alkali- oder Erdalkalimetalls umsetzt. Y und Z bedeuten bevorzugt Br oder Cl und besonders bevorzugt Cl.

Die Verbindungen der Formel C können durch Amidierung bzw. Transamidierung der entsprechenden Carbonsäureester, -amide oder -halogenide hergestellt werden. Die Bildung von Carbonsäureamiden aus Carbonsäureestern und Aminen in Gegenwart von Trialkylaluminium oder Dialkylaluminiumhalogenid, zum Beispiel mit Trimethylaluminium oder Dimethylaluminiumchlorid, ist von S. M. Weinreb in Organic Syntheses, 59, Seiten 49-53 (1980) beschrieben. Die Carbonsäureester sind erhältlich durch die Umsetzung von trans-1,3-Dihalogenpropen (zum Beispiel trans-1,3-Dichlorpropen) mit entsprechenden Carbonsäureestern in Gegenwart starker Basen, zum Beispiel Alkalimetallamiden.

Die Verbindungen der Formel B können durch Halogenierung, in an sich bekannter Weise, beispielweise nach dem von J. Maibaum in Tetrahedron Letters, Vol. 41 (2000), Seiten 10085-10089 beschriebenen Verfahren, aus 2-Alkyl-3-heterocyclyl-1-propanolen der Formel D hergestellt werden. Die praktisch enantiomerenreinen 2-Alkyl-3-heterocyclyl-1-propanole der Formel D können durch enantioselektive Hydrierung der entsprechenden (E)-Allylalkohole hergestellt werden.

Hierzu werden die (E)-2-Alkyl-3-heterocyclyl-prop-2-en-1-ole der untenstehenden Formel I in Gegenwart von Wasserstoff und katalytischen Mengen eines Metallkomplexes als asymmetrischer Hydrierkatalysator, der Metalle aus der Gruppe Ruthenium, Rhodium oder Iridium enthält, an die chirale zweizähnige Liganden gebunden sind, zu einer Verbindung der Formel D hydriert.

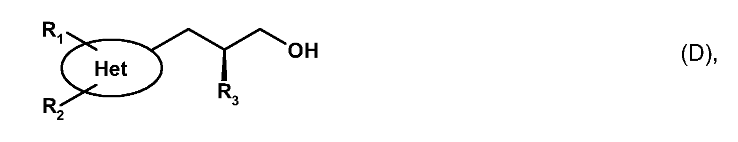

Die asymmetrischen Hydrierungen der (E)-2-Alkyl-3-heterocyclyl-prop-2-en-1-ole der untenstehenden Formel I mit homogenen, asymmetrischen Hydrierkatalysatoren sind an sich bekannt und zum Beispiel von J. M. Brown in E. Jacobsen, A. Pfaltz, H. Yamamoto (Eds.) in Comprehensive Asymmetric Catalysis I bis III, Springer Verlag, 1999, Seiten 121-182 und von X. Zhang in Chemical Reviews, Vol 103 (2003), Seiten 3029-3069 beschrieben. Besonders wirksam sind Ruthenium-, Rhodium- und Iridiumkatalysatoren.

Asymmetrische Hydrierungen der (E)-2-Alkyl-3-heterocyclyl-prop-2-en-1-ole der untenstehenden Formel I können bevorzugt unter Verwendung von Ruthenium-, Iridium- und Rhodiumkatalysatoren, wie zum Beispiel von M. Bänziger und T. Troxler in Tetrahedron: Asymmetry, Vol. 14 (2003) Seiten 3469-3477, R. Gilbertson in Tetrahedron Letters, Vol. 44 (2003) Seiten 953-955, P. G. Andersson im Journal of the American Chemical Society, Vol. 126 (2004), Seiten 14308-14309, A. Pfaltz im Organic Letters, Vol. 6 (2004), Seiten 2023-2026, und F. Spindler in Tetrahedron: Asymmetry, Vol. 15 (2004) Seiten 2299-2306 beschrieben, durchgeführt werden.

Als Liganden für Rhodium und Ruthenium werden häufig chirale ditertiäre Bis-phosphine verwendet. Solche chiralen ditertiären Bis-phosphine sind zum Beispiel von X. Zhang in Chemical Reviews, Vol 103 (2003), Seiten 3029-3069 beschrieben.

Als Liganden für Iridium werden häufig chirale Phosphin-oxazolin-Liganden verwendet. Solche chiralen Phosphin-oxazolin-Liganden sind zum Beispiel von A. Pfaltz im Advanced Synthesis and Catalysis, Vol. 345 (2003), Seiten 33-43 beschrieben.

Es wurde nun überraschend gefunden, dass man (E)-Allylalkohole der Formel I, welche ein Schlüsselzwischenprodukt für das eingangs erwähnte neue Herstellungsverfahren ausgehend von 2,7-Dialkyl-8-heterocyclyl-4-octenoylamiden sind, in nur zwei Verfahrensstufen in hohen Ausbeuten herstellen kann: Wenn man geeignet substituierte, ungesättigte Heterocyclyl-Bromide, -lodide oder auch -Triflate, -Tosylate oder -Mesylate mit Prop-2-in-1-ol zu 3-Heterocyclyl-prop-2-in-1-olen umsetzt, fallen die Produkte in hohen Ausbeuten an. Die 3-Heterocyclyl-prop-2-in-1-ole sind ein wichtiges Zwischenprodukt des Verfahrens. Ausgehend von diesen 3-Heterocyclyl-prop-2-in-1-olen lassen sich die (E)-2-Alkyl-3-heterocyclyl-prop-2-en-1-ole in hohen Ausbeuten durch Umsetzung mit einer Alkylmetallverbindung erhalten.

Zur ersten Verfahrensstufe ähnliche Verfahren für 1-Aryl-prop-2-in-Verbindungen werden in der Literatur von S. Bhattacharya und S. Sengupta in Tetrahedron Letters, Vol. 45 (2004), Seiten 8733-8736 beschrieben. Weiterhin werden zur zweiten Verfahrensstufe ähnliche Verfahren für 2-Alkyl-3-phenyl-prop-2-en-1-ol-Verbindungen in der Literatur von J. G. Duboudin und B. Jousseaume im Journal of Organometallic Chemisty, Vol. 168 (1979), Seiten 1-11 bzw. A. Claesson in Tetrahedron Letters, Vol. 15 (1974), Seiten 2161-2162 beschrieben.

Im Vergleich zu dem aus der WO 2005/090305 A1 bekannten Verfahren zur Herstellung von δ-Amino-γ-hydroxy-ω-heterocyclyl-alkancarbonsäureamiden zeichnet sich dieses neue Verfahren dadurch aus, dass alle beteiligten Reaktionsschritte mit guten Ausbeuten durchgeführt werden können und dass Reaktionsschritte vermieden werden, die bei Temperaturen unter - 40°C durchgeführt werden müssen, was für die Herstellung im industriellen Maßstab von praktischem und finanziellem Vorteil ist..

Gegenstand der Erfindung ist ein Verfahren zur Herstellung von Verbindungen der Formel I, 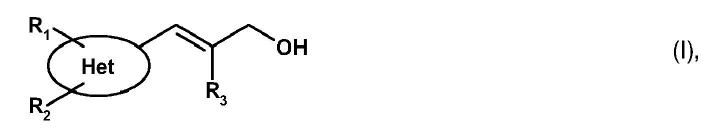 worin Het ein bicyclisches, über ein C-Atom an das Restmolekül geknüpftes, ungesättigtes Heterocyclyl bedeutet, wobei der nicht direkt an das Restmolekül gebundene Ring durch R₁ und R₂ substituiert ist, R₁ und R₂ unabhängig voneinander H, C₁-C₈-Alkyl, Halogen, Polyhalogen-C₁-C₈-alkoxy, Polyhalogen-C₁-C₈-alkyl, C₁-C₈-Alkoxy, C₁-C₈-Alkoxy-C₁-C₈-alkyl, oder C₁-C₈-Alkoxy-C₁-C₈-alkoxy darstellen, wobei R₁ und R₂ nicht gleichzeitig H bedeuten, und R₃ C₁-C₈-Alkyl bedeutet, das dadurch gekennzeichnet ist, dass man
a) eine Verbindung der Formel II 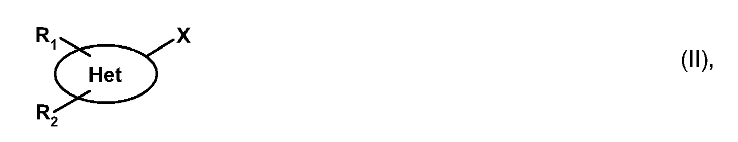 worin Het, R₁ und R₂ die zuvor angegebenen Bedeutungen haben und X Br, 1, Triflat, Tosylat oder Mesylat darstellt, mit Prop-2-in-1-ol zu einer Verbindung der Formel III umsetzt, 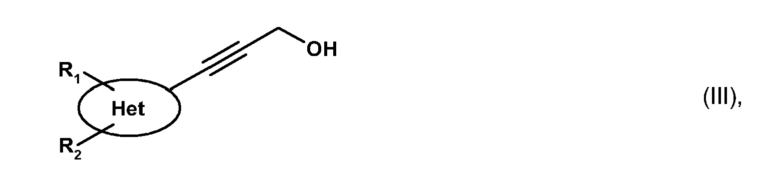
b) die Verbindung der Formel III mit einer Alkylmetallverbindung, worin "Alkyl" die zuvor für R₃ angegebene Bedeutung hat, zu einer Verbindung der Formel I umsetzt.

R₁ und R₂ können als C₁-C₈-Alkyl linear oder verzweigt sein und bevorzugt 1 bis 4 C-Atome enthalten. Beispiele sind Methyl, Ethyl, n- und i-Propyl, n-, i- und t-Butyl, Pentyl und Hexyl.

R₁ und R₂ können als Polyhalogen-C₁-C₈-alkyl linear oder verzweigt sein und bevorzugt 1 bis 4, besonders bevorzugt 1 oder 2 C-Atome enthalten. Beispiele sind Fluormethyl, Difluormethyl, Trifluormethyl, Chlormethyl, Dichlormethyl, Trichlormethyl, 2-Chlorethyl und 2,2,2-Trifluorethyl.

R₁ und R₂ können als Polyhalogen-C₁-C₈-alkoxy linear oder verzweigt sein und bevorzugt 1 bis 4, besonders bevorzugt 1 oder 2 C-Atome enthalten. Beispiele sind Fluormethoxy, Difluormethoxy, Trifluormethoxy, Chlormethoxy, Dichlormethoxy, Trichlormethoxy, 2-Chlorethoxy und 2,2,2-Trifluorethoxy.

R₁ und R₂ können als Halogen, einschliesslich Halogen in Polyhalogen-C₁-C₈-alkyl und Polyhalogen-C₁-C₈-alkoxy, F, Cl oder Br bedeuten, wobei F und Cl bevorzugt sind.

R₁, R₂ und R₅ können als C₁-C₈-Alkoxy linear oder verzweigt sein und bevorzugt 1 bis 4 C-Atome enthalten. Beispiele sind Methoxy, Ethoxy, n- und i-Propoxy, n-, i- und t-Butoxy, Pentoxy und Hexoxy.

R₁ und R₂ können als C₁-C₈-Alkoxy-C₁-C₈-alkyl linear oder verzweigt sein. Die Alkoxygruppe enthält bevorzugt 1 bis 4 und besonders 1 oder 2 C-Atome, und die Alkylgruppe enthält bevorzugt 1 bis 4 C-Atome. Beispiele sind Methoxymethyl, 1-Methoxyeth-2-yl, 1-Methoxy-prop-3-yl, 1-Methoxybut-4-yl, Methoxypentyl, Methoxyhexyl, Ethoxymethyl, 1-Ethoxyeth-2-yl, 1-Ethoxyprop-3-yl, 1-Ethoxybut-4-yl, Ethoxypentyl, Ethoxyhexyl, Propoxymethyl, Butoxymethyl, 1-Propoxyeth-2-yl und 1-Butoxyeth-2-yl.

R₁ und R₂ können als C₁-C₈-Alkoxy-C₁-C₈-alkoxy linear oder verzweigt sein. Die Alkoxygruppe enthält bevorzugt 1 bis 4 und besonders 1 oder 2 C-Atome, und die Alkoxygruppe enthält bevorzugt 1 bis 4 C-Atome. Beispiele sind Methoxymethoxy, 2-Methoxyethoxy, 3-Methoxypropoxy, 4-Methoxybutoxy, Methoxypentoxy, Methoxyhexoxy, Ethoxymethoxy, 2-Ethoxyethoxy, 3-Ethoxypropoxy, 4-Ethoxybutoxy, Ethoxypentoxy, Ethoxyhexoxy, Propoxymethoxy, Butoxymethoxy, 2-Propoxyethoxy und 2-Butoxyethoxy.

In einer bevorzugten Ausführungsform bedeutet R₁ Methoxy- oder Ethoxy-C₁-C₄-alkyl, und R₂ stellt bevorzugt Methyl, Ethyl, Methoxy oder Ethoxy dar. Ganz besonders bevorzugt sind Verbindungen der Formel I, worin R₁ 3-Methoxy-propyl oder 4-Methoxy-butyl und R₂ Methyl oder Methoxy bedeuten.

R₃, R₄, R₅ und R₆ können als C₁-C₈-Alkyl linear oder verzweigt sein und bevorzugt 1 bis 4 C-Atome enthalten. Beispiele sind Methyl, Ethyl, n- und i-Propyl, n-, i- und t-Butyl, Pentyl und Hexyl. In einer bevorzugten Ausführungsform stellt in den Verbindungen der Formel I R₃ Isopropyl dar.

Het kann als bicyclisches, über ein C-Atom an das Restmolekül geknüpftes, ungesättigtes Heterocyclyl bicyclische, ungesättigte heterocyclische Reste mit 1 bis 4 Stickstoff- und/oder 1 oder 2 Schwefel- oder Sauerstoffatomen umfassen, wobei Reste mit einem oder 2 Stickstoffatomen bevorzugt sind. Bevorzugte Bicyclen bestehen aus jeweils 5- und/oder 6-gliedrigen Ringen. Beispiele für Het sind Benzothiazolyl, Chinazolinyl, Chinolyl, Chinoxalinyl, Isochinolyl, Benzo[b]thienyl, Isobenzofuranyl, Benzoimidazolyl, Indolyl, Dihydrobenzofuranyl, Tetrahydro-chinoxalinyl, 3,4-Dihydro-2H-benzo[1,4]oxazinyl, 1 H-Pyrrolizinyl, Phthalazinyl, Dihydro-2H-benzo[1,4]thiazinyl, 1 H-Pyrrolo[2,3-b]pyridyl, Imidazo[1,5-a]pyridyl, Benzooxazolyl, 2,3-Dihydroindolyl, Indazolyl oder Benzofuranyl. Het bedeutet besonders bevorzugt 1H-Indol-6-yl, Imidazo[1,5-a]pyridin-6-yl oder 1H-Indazol-6-yl.

Besonders bevorzugt sind Verbindungen der Formel I, worin mit R₁ und R₂ substituiertes Het 1-(3-Methoxy-propyl)-3-methyl-1 H-indol-6-yl, 3-(3-Methoxy-propyl)-1-methyl-imidazo[1,5-a]pyridin-6-yl oder 1-(3-Methoxy-propyl)-3-methyl-1 H-indazol-6-yl und R₃ Isopropyl darstellen.

Die in der Verfahrensstufe a) verwendeten Ausgangsverbindungen der Formel II (X = Br) sind aus der WO 2005/090305 A1 bekannt. Die Ausgangsverbindungen der Formeln II (X = I) sind bekannt oder können analog zu bekannten Verfahren hergestellt werden. Die Ausgangsverbindungen der Formeln II (X = Triflat, Tosylat, Mesylat) sind analog zu bekannten Verfahren aus den entsprechenden Alkoholen erhältlich.

Die Verfahrensstufe a) wird vorteilhaft bei höheren Temperaturen, zum Beispiel 60 bis 90°C, in Gegenwart katalytischer Mengen eines Palladium Katalysators wie beispielsweise Pd(PPh₃)₄, Pd(PPh₃)₂Cl₂ oder Pd/C + PPh₃, sowie gegebenenfalls katalytischer Mengen eines Kupfer(I) Halids wie beispielsweise Cul und in Gegenwart wenigstens äquivalenter Mengen einer Amin-Base durchgeführt. Die Reaktion wird ferner zweckmässig ohne Lösungsmittel durchgeführt, kann allerdings auch in polaren, protischen Lösungsmitteln wie beispielsweise Wasser durchgeführt werden. Geeignete Amin-Basen sind zum Beispiel Piperidin, Pyrrolidin, Diisopropyethylamin, Triethylamin oder Ammoniak.

Die Addition gemäss Verfahrensstufe b) wird vorteilhaft bei tieferen Temperaturen, zum Beispiel-10°C bis Raumtemperatur (z.B. 23°C), in Gegenwart katalytischer Mengen eines Kupfer(1) Halids wie beispielsweise Cul durchgeführt. Die Alkylmetallverbindung wird vorteilhaft im Überschuss eingesetzt. Die Reaktion kann gegebenenfalls in Gegenwart eines Zusatzes wie z.B. TMEDA durchgeführt werden. Die Reaktion wird ferner zweckmässig in einem Lösungsmittel durchgeführt, wobei Ether wie zum Beispiel Diethylether, Tetrahydrofuran und Dioxan besonders geeignet sind.

Bevorzugte Alkylmetallverbindungen sind Alkylmagnesium-Halide, wobei "Halid" Br oder Cl bedeutet.

Die Reaktion der Verfahrensstufe b) verläuft regiospezifisch und führt nahezu ausschliesslich zu den gewünschten Produkten der Formel 1.

Mit dem erfindungsgemässen, regiospezifischen Verfahren können die Zwischenprodukte zur Herstellung der Verbindung der Formel (B) über alle Verfahrensstufen in hohen Ausbeuten hergestellt werden. Die hohen Gesamtausbeuten machen das Verfahren für den industriellen Einsatz geeignet.

Gegenstand der Erfindung sind auch die Verbindungen (Zwischenprodukte) der Formel III, 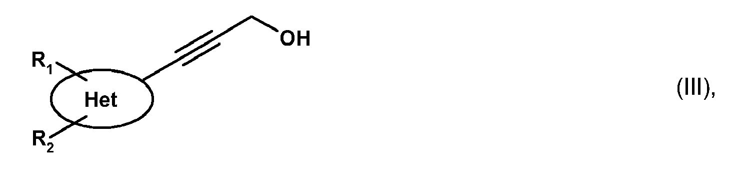 worin Het, R₁ und R₂ die zuvor angegebenen Bedeutungen haben.

Für Het, R₁ und R₂ gelten die zuvor beschriebenen Ausführungsformen und Bevorzugungen.

Die nachfolgenden Beispiele erläutern die Erfindung näher.

HPLC-Gradient auf Hypersil BDS C-18 (5 µm); Säule: 4 x 125 mm
(I) 90% Wasser*/10% Acetonitril* zu 0% Wasser*/100% Acetonitril* in 5 Minuten + 2.5 Minuten (1.5 ml/min)
(II) 95% Wasser*/5% Acetonitril* zu 0% Wasser*/100% Acetonitril* in 40 Minuten (0.8 ml/min)
* enthält 0.1 % Trifluoressigsäure

### Beispiel A)

### Verfahren zur Herstellung von 2-[1-[1-(3-Methoxy-propyl)-3-methyl-1 H-indazol-6-yl]-meth-E)-yliden]-3-methyl-butan-1-ol (A2)

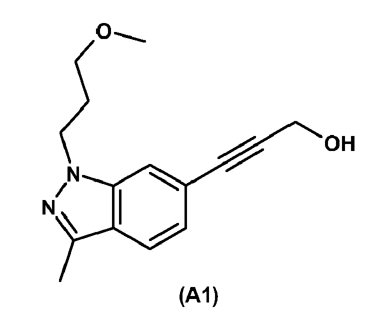

### Beispiel A1:

### Herstellung von 3-[1-(3-Methoxy-propyl)-3-methyl-1 H-indazol-6-yl]-prop-2-in-1-ol

In einem 250 ml Rundkolben werden 17.481 mmol 6-Brom-1-(3-methoxy-propyl)-3-methyl-1 H-indazol [865156-81-6] und 0.699 mmol Tetrakistriphenylphosphin Palladium(0) vorgelegt, die Apparatur wird 30 Minuten am Hochvakuum evakuiert und anschliessend mit Argon begast. In das Reaktionsgefäss werden 40 ml Pyrrolidin und 34.962 mmol Prop-2-in-1-ol zugegeben und die Lösung wird während 5 Stunden bei 80°C gerührt. Die Reaktionsmischung wird auf Raumtemperatur abgekühlt und auf 200 ml gesättigte, wässrige Ammoniumchlorid-Lösung gegossen. Es wird mit Essigester (2 x 250 ml) extrahiert. Die vereinigten organischen Phasen werden Sole (250 ml) gewaschen, mit Natriumsulfat getrocknet, filtriert und am Rotationsverdampfer eingedampft. Aus dem Rückstand wird mittels Flashchromatographie (SiO₂ 60F, Essigester-Hexan 2:1) die Titelverbindung A1 als oranges Oel erhalten (4.14 g). Rf = 0.26 (Essigester-Heptan 2:1); Rt = 3.42 (Gradient I).

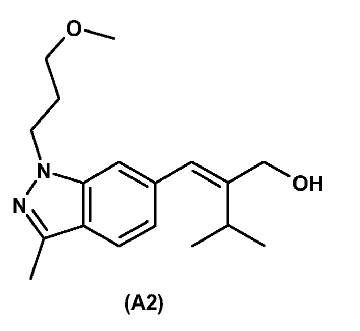

### Beispiel A2:

### Herstellung von 2-[1-[1-(3-Methoxy-propyl)-3-methyl-1 H-indazol-6-yl]-meth-(E)-yliden] -3-methyl-butan-1-ol

Ein getrocknetes 20 ml Schlenk-Rohr wird unter Argon Atmosphäre mit einer Lösung von 2.078 mmol 3-[1-(3-Methoxy-propyl)-3-methyl-1 H-indazol-6-yl]-prop-2-in-1-ol in 5 ml Tetrahydrofuran befüllt. Die Lösung wird mit 0.208 mmol festem Kupfer(I) iodid versetzt und die Suspension wird auf-10°C abgekühlt. Anschliessend werden 2.60 ml einer 2.0 molaren Isopropylmagnesiumchlorid-Lösung in Diethylether zugegeben. Die entstehende Mischung wird während 1.5 Stunden bei -10°C gerührt. Dann wird das Reaktionsgemisch auf gesättigte, wässrige Ammoniumchlorid-Lösung bei 0°C gegossen und anschliessend mit Wasser und Essigester verdünnt. Die Phasen werden getrennt, die wässrige Phase wird mit Essigester (2 x) extrahiert. Die vereinigten organischen Phasen werden mit Sole gewaschen, mit Natriumsulfat getrocknet, filtriert und am Rotationsverdampfer eingedampft. Aus dem Rückstand wird mittels Flashchromatographie (SiO₂ 60F, Essigester-Hexan 2:1) die Titelverbindung A2 als gelbes Oel erhalten (485 mg). Rf = 0.37 (Essigester-Heptan 2:1); Rt = 4.00 (Gradient I).

### Beispiel B)

### Verfahren zur Herstellung von 2-[1-[1-(3-Methoxy-propyl)-3-methyl-1 H-indol-6-yl]-meth-(E)-yliden]-3-methyl-butan-1-ol (B2)

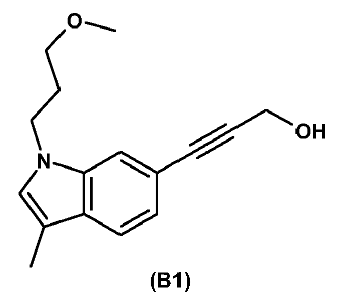

### Beispiel B1:

### Herstellung von 3-[1-(3-Methoxy-propyl)-3-methyl-1 H-indol-6-yl]-prop-2-in-1-ol

In einem 250 ml Rundkolben werden 15.717 mmol 6-Brom-1-(3-methoxy-propyl)-3-methyl-1 H-indol (WO 2005/090305 A1) und 0.629 mmol Tetrakistriphenylphosphin Palladium(0) vorgelegt, die Apparatur wird 30 Minuten am Hochvakuum evakuiert und anschliessend mit Argon begast. In das Reaktionsgefäss werden 40 ml Pyrrolidin und 31.434 mmol Prop-2-in-1-ol zugegeben und die Lösung wird während 21 Stunden bei 80°C gerührt. Die Reaktionsmischung wird auf Raumtemperatur abgekühlt und auf 200 ml gesättigte, wässrige Ammoniumchlorid-Lösung gegossen. Es wird mit Essigester (2 x 250 ml) extrahiert. Die vereinigten organischen Phasen werden Sole (250 ml) gewaschen, mit Natriumsulfat getrocknet, filtriert und am Rotationsverdampfer eingedampft. Aus dem Rückstand wird mittels Flashchromatographie (SiO₂ 60F, Essigester-Hexan 2:1) die Titelverbindung B1 als gelbes Oel erhalten (3.40 g). Rf = 0.41 (Essigester-Heptan 2:1); Rt = 4.20 (Gradient I).

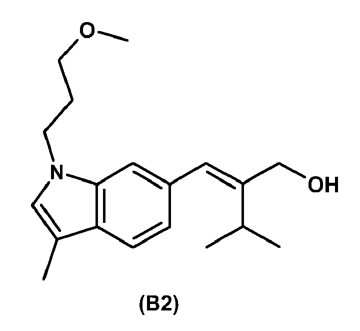

### Beispiel B2:

### Herstellung von 2-[1-[1-(3-Methoxy-propyl)-3-methyl-1 H-indol-6-yl]-meth-(E)-yliden]-3-methyl-butan-1-ol

Ein getrocknetes 10 ml Schlenk-Rohr wird unter Argon Atmosphäre mit einer Lösung von 1.00 mmol 3-[1-(3-Methoxy-propyl)-3-methyl-1 H-indol-6-yl]-prop-2-in-1-ol in 2.5 ml Tetrahydrofuran befüllt. Die Lösung wird mit 0.10 mmol festem Kupfer(I) iodid versetzt und die Suspension wird auf-10°C abgekühlt. Anschliessend werden 1.25 ml einer 2.0 molaren Isopropylmagnesiumchlorid-Lösung in Diethylether zugegeben. Die entstehende Mischung wird während 18 Stunden bei -18°C gerührt. Dann wird das Reaktionsgemisch auf gesättigte, wässrige Ammoniumchlorid-Lösung bei 0°C gegossen und anschliessend mit Wasser und Essigester verdünnt. Die Phasen werden getrennt, die wässrige Phase wird mit Essigester (2 x) extrahiert. Die vereinigten organischen Phasen werden mit Sole gewaschen, mit Natriumsulfat getrocknet, filtriert und am Rotationsverdampfer eingedampft. Aus dem Rückstand wird mittels Flashchromatographie (SiO₂ 60F, Essigester-Hexan 2:1) die Titelverbindung B2 als gelbes Oel erhalten (214 mg). Rf = 0.45 (Essigester-Heptan 2:1); Rt = 4.84 (Gradient I).

## Patentansprüche

1. Verfahren zur Herstellung einer Verbindung der Formel I 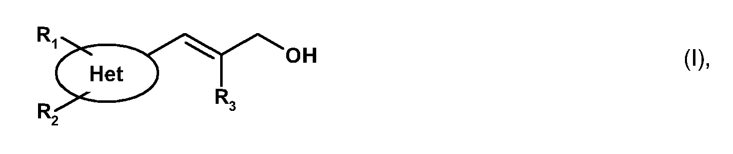 worin Het ein bicyclisches, über ein C-Atom an das Restmolekül geknüpftes, ungesättigtes Heterocyclyl bedeutet, wobei der nicht direkt an das Restmolekül gebundene Ring durch R₁ und R₂ substituiert ist, R₁ und R₂ unabhängig voneinander H, C₁-C₈-Alkyl, Halogen, Polyhalogen-C₁-C₈-alkoxy, Polyhalogen-C₁-C₈-alkyl, C₁-C₈-Alkoxy, C₁-C₈-Alkoxy-C₁-C₈-alkyl, oder C₁-C₈-Alkoxy-C₁-C₈-alkoxy darstellen, wobei R₁ und R₂ nicht gleichzeitig H bedeuten, und R₃ C₁-C₈-Alkyl bedeutet, das **dadurch gekennzeichnet ist, dass** man
a) eine Verbindung der Formel II 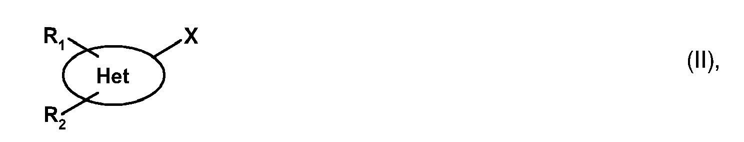 worin Het, R₁ und R₂ die zuvor angegebenen Bedeutungen haben und X Br, I, Triflat, Tosylat oder Mesylat darstellt, mit Prop-2-in-1-ol zu einer Verbindung der Formel III umsetzt, 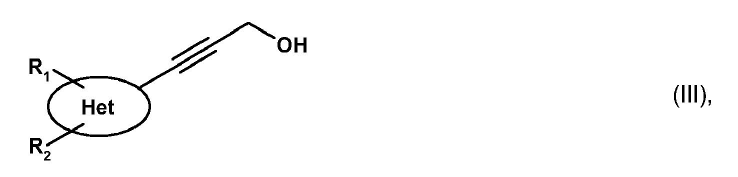
b) die Verbindung der Formel III mit einer Alkylmetallverbindung, worin "Alkyl" die zuvor für R₃ angegebene Bedeutung hat, zu einer Verbindung der Formel I umsetzt.

2. Verfahren gemäss Anspruch 1 zur Herstellung einer Verbindung der Formel I, wobei R₁ Methoxy- oder Ethoxy-C₁-C₄-alkyl, und R₂ Methyl, Ethyl, Methoxy oder Ethoxy darstellt.

3. Verfahren gemäss einem der Ansprüche 1 oder 2 zur Herstellung einer Verbindung der Formel 1, wobei Het einen bicyclischen, ungesättigten heterocyclischen Rest mit 1 bis 4 Stickstoff- und/oder 1 oder 2 Schwefel- oder Sauerstoffatomen, bestehend aus jeweils 5-und/oder 6-gliedrigen Ringen, darstellt.

4. Verfahren gemäss einem der Ansprüche 1 bis 3 zur Herstellung einer Verbindung der Formel I, wobei mit R₁ und R₂ substituiertes Het 1-(3-Methoxy-propyl)-3-methyl-1 H-indol-6-yl, 3-(3-Methoxy-propyl)-1-methyl-imidazo[1,5-a]pyridin-6-yl oder 1-(3-Methoxy-propyl)-3-methyl-1 H-indazol-6-yl und R₃ Isopropyl darstellt.

5. Verbindung der Formel III, 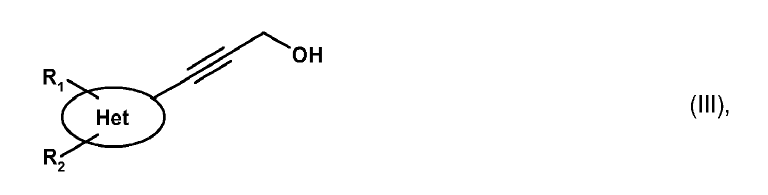 worin Het ein bicyclisches, über ein C-Atom an das Restmolekül geknüpftes, ungesättigtes Heterocyclyl bedeutet, wobei der nicht direkt an das Restmolekül gebundene Ring durch R₁ und R₂ substituiert ist, R₁ und R₂ unabhängig voneinander H, C₁-C₈-Alkyl, Halogen, Poly-halogen-C₁-C₈-alkoxy, Polyhalogen-C₁-C₈-alkyl, C₁-C₈-Alkoxy, C₁-C₈-Alkoxy-C₁-C₈-alkyl, oder C₁-C₈-Alkoxy-C₁-C₈-alkoxy darstellen, wobei R₁ und R₂ nicht gleichzeitig H bedeuten.

6. Verfahren zur Herstellung einer Verbindung der Formel A 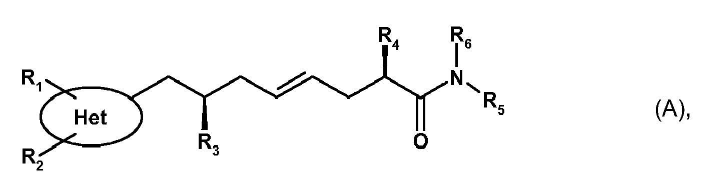 worin Het ein bicyclisches, über ein C-Atom an das Restmolekül geknüpftes, ungesättigtes Heterocyclyl bedeutet, wobei der nicht direkt an das Restmolekül gebundene Ring durch R₁ und R₂ substituiert ist, R₁ und R₂ unabhängig voneinander H, C₁-C₈-Alkyl, Halogen, Polyhalogen-C₁-C₈-alkoxy, Polyhalogen-C₁-C₈-alkyl, C₁-C₈-Alkoxy, C₁-C₈-Alkoxy-C₁-C₈-alkyl, oder C₁-C₈-Alkoxy-C₁-C₈-alkoxy darstellen, wobei R₁ und R₂ nicht gleichzeitig H bedeuten, R₃ C₁-C₈-Alkyl bedeutet, R₄ für C₁-C₈-Alkyl steht, R₅ C₁-C₈-Alkyl oder C₁-C₈-Alkoxy bedeutet, R₆ C₁-C₈-Alkyl darstellt, oder R₅ und R₆ zusammen gegebenenfalls mit C₁-C₄-Alkyl, Phenyl oder Benzyl substituiertes Tetramethylen, Pentamethylen, 3-Oxa-1,5-pentylen oder -CH₂CH₂O-C(O)- sind, **dadurch gekennzeichnet, dass** man eine Verbindung der Formel B 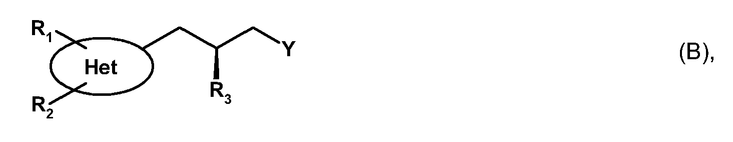 mit einer Verbindung der Formel C, 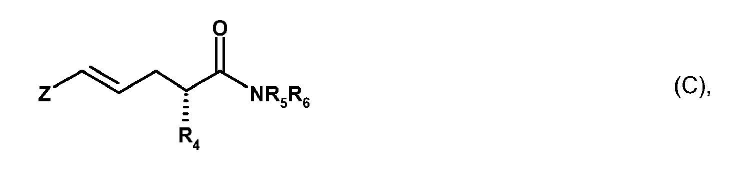 worin Het und R₁ bis R₆ die zuvor angegebenen Bedeutungen haben, Y Cl, Br oder I und Z Cl, Br oder I bedeuten, in Gegenwart eines Alkali- oder Erdalkalimetalls umsetzt,
wobei die Verbindung der Formel B durch Halogenierung einer Verbindung der Formel D erhalten wird, 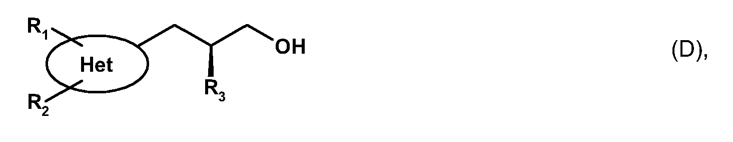 welche durch asymmetrische Hydrierung einer gemäss Anspruch 1 hergestellten Verbindung der Formel I hergestellt wird.
